# EUROPEAN PATENT SPECIFICATION

(11) **EP 1 817 588 B1**
(45) Date of publication and mention of the grant of the patent: **03.11.2010**
(21) Application number: 05849230.7
(22) Date of filing: 15.11.2005
(51) Int. Cl.: G01N 33/558, G01N 33/76, G01N 33/569

(54) **LATERAL-FLOW TEST DEVICE PROVIDING IMPROVED TEST RESULT VALIDITY**
SEITENSTROM-TESTVORRICHTUNG MIT VERBESSERTER TESTERGEBNIS-GÜLTIGKEIT
DISPOSITIF D'ESSAIE À ÉCOULEMENT LATÉRAL DÉSTINÉ À DONNER DES RESULTATS DE VALIDITÉ AMÉLIORÉE

(30) Priority: 01.12.2004 EP 04028390
(43) Date of publication of application: 15.08.2007
(73) Proprietor: AraGen Biotechnology Co. Ltd., 11710 Naor (JO)
(72) Inventor: MOHAMMED, Murshed AbdelQader, 11118 Amman (JO)
(74) Representative: Winkler, Andreas Fritz Ernst
(86) International application number: PCT/EP2005/012215
(87) International publication number: WO 2006/058610

(56) References cited:
- EP-A- 1 376 131
- WO-A-98/22824
- GB-A- 2 383 130
- US-A- 5 602 040
- US-A- 5 714 341
- US-A1- 2002 001 852

## Description

The present invention refers to a lateral-flow test device providing improved test result validity comprising at least one indication means indicating whether a test result will be inconclusive and at least one indication means indicating that a test result is ready for read. The present invention further refers to a use of such a lateral-flow test device.

### Background of the invention

In recent years the *in vitro* diagnostics (IVD) industry has made enormous efforts to develop membrane-based lateral-flow tests. Such tests have found applications in both clinical and non-clinical fields (ref. 1). A clinical utility of this test format has been shown for more than 150 different analytes, and many of them are target now of commercially available diagnostic products (ref. 3). The wide range of applications for such devices has been reviewed (refs. 1, 2).

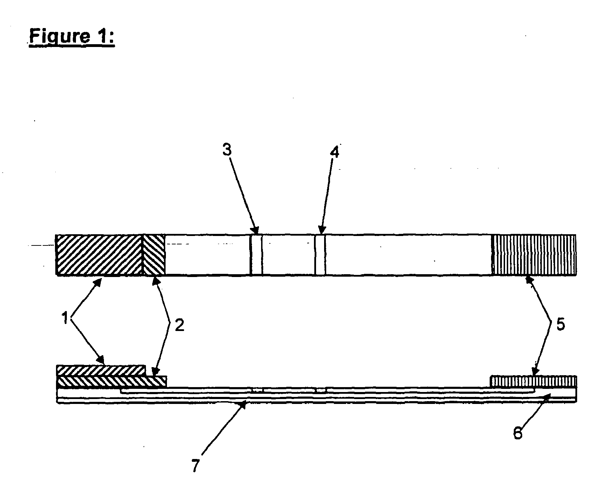

Membrane-based lateral-flow test devices, e.g. rapid-flow immunochromatographic test devices, are made up of a number of components commonly including a sample pad, a conjugate pad, a membrane that incorporates capture reagents, and an absorbent pad (see *Figure 1*). In practice, the user dispenses a patient sample (usually urine or whole blood) onto the sample pad. The sample then flows through the sample pad into the conjugate pad where it mixes with and releases the detector reagent. This mixture then flows across the membrane where it binds with the test and control reagents. When the mixture binds to the reagent that forms the test line, a positive result is indicated. The color intensity of the test line is proportional to the concentration of analyte in the sample. Excess sample that flows beyond the test and control lines is taken up in the absorbent pad.

Membrane-based lateral-flow test devices for diagnostic purposes are easy to operate and thus do not only contribute to the comfort of professional users, e.g. medical stuff, but also allow the operation by non-professionals users, e.g. most patients.

However, one serious problem associated with the use of membrane-based lateral-flow test devices is atmospheric humidity. Most of the rapid test components are extremely hygroscopic, especially nitrocellulose membrane which absorbs moisture strongly and equilibrates with ambient conditions within seconds. As a consequence, false positive (or negative) results may be produced. Thus, unfavorable storage and/or use conditions due to high relative humidity potentially affects the validity of the test result. Therefore, it would be of advantage to have a lateral-flow test device available indicating whether the test result will be inconclusive.

GB 2 383 130 discloses a test device, e.g. in form of a test strip, for detecting the presence of drugs within drinks. For that purpose, the test device comprises a pad that is capable of accepting a liquid by capillary action and a second pad soaked in a chemical agent, e.g. a gold conjugate and an antibody, capable of detecting the substance being tested for. In case of using antibody technology, the test strip further comprises a reaction membrane including a line of a test antibody which detects the antibody-gold conjugate complex. Preferably, the second pad additionally contains a chemical agent, e.g. a control gold conjugate and a control antibody, and a control line forms to show that the constituents of the test are working correctly. In one embodiment, the test strip has a moisture detector region which changes colour if the test device is wet or has been spoilt by moisture or usage.

WO 98/22824 discloses a test strip for detecting the presence of an analyte of interest in a biological liquid sample. The test strip has a control zone comprising a coloured material, e.g. a blue cobalt salt, capable of being washed away on contact with a liquid sample as the sample migrates across the control zone. Thereby, the user is conclusively informed that the strip is functioning normally. Moreover, use of hygroscopic colours for the control zone will enable the indication of any exposure to humidity prior to use, since as a result of such exposit, the colour in the control zone will change, e.g. from blue in the dry form to brown in the hydrous form. The analyte of interest can be an antibody, e.g. directed against HIV-1 or HIV-2, a hormone, e.g. hCG, or a microorganism.

US 5,714,341 discloses a device for determining amylase in saliva using chromogenic substrate effective to produce a coloured product upon reaction. It is further disclosed that after passing the reaction region, the sample passes through a detection region comprising a hydration indicator substance, e.g. copper sulphate or cobalt chloride. When hydration of the detection region is complete, the reaction region is inspected for determining the test result. An exemplary analyte to be detected is an immunoglobulin specific for HIV-1 or HIV-2.

US 5,602,040 discloses an analytical test device useful, for example, in pregnancy testing. For that purpose, the analyte can be hCG. If present, a control zone can be designed merely to convey an unrelated signal to the user that the device has worked. Alternatively, the control zone can contain an anhydrous reagent that, when moistened, produces a colour change or colour formation, e.g. anhydrous copper sulphate.

EP 1 376 131 discloses an assay device comprising a sample presence signal generation means which is not generated by means of an immunoreaction on which the analyte detection is based. In one embodiment, the signal generation means comprises a colour changing material which undergoes a change in its visible properties upon wetting. The resulting coloured material provides a visible indication that sufficient liquid sample has been taken up, i.e. that the detection reaction can function correctly. Preferably, the assay device comprises a control signal generation means downstream of the analyte detection region adapted to generate a control signal indicative that one or more reagents present in the assay device are functioning. Finally, a local separation of indication windows is disclosed.

Another problem is that the user must know when the analysis reaction indicating the presence or absence of an analyte is completed and thus the result is ready for read. In all currently available rapid-flow immunochromatographic test devices in the IVD market around the globe, the user is provided with instructions, i.e. by the package insert, such as: "Please read results after x minutes". Thus, the final consumer will be forced to use a timer or a clock, and the need of additional equipment may be more or less uncomfortable. A more serious problem, however, is the risk of inaccurate determination of time since such external time measurement does not have any relationship with the real analysis reaction running at the test device. Reading of result at a time when the analysis reaction is not yet completed or even when the reaction products are already disintegrating potentially produce false results. Therefore, it would be of advantage to have a lateral-flow test device available comprising an internal control with respect to the reading time. US 2002/001852 discloses an assay device for determining an analyte in an aqueous sample comprising a timer function. For that purpose, a time indicator is placed downstream of a detection zone for indicating when liquid applied to a liquid application zone has reached the time indicator by a visible colour change when hydrated by the aqueous sample. An exemplary indicator substance is cobalt dichloride hexahydrate. Furthermore, the indicator substance capable of hydration may also serve as a test that the assay device is viable, since in case of leakage of moisture into the device, which reduces the shelf-life of the device, the indicator substance will change colour. The timer function of the assay device disclosed in US 2002/001852 is based on a colour change due to hydration by the aqueous sample. In other words, the time of colour change depends on that time the sample needs to migrate a predetermined distance, and thus the colour change will occur at a predetermined time after sample application. This concept is also known from US 5,714,341 (see above) disclosing that the test result is to be read when colour change of the detection region indicates complete hydration. Thus, similar to an external time control, the configurations disclosed in US 2002/001852 and US 5,714,341 do not provide any internal relationship to the real analysis reaction and, furthermore, do not allow any flexibility in the result reading time which strongly depends on the nature of the reaction.

Therefore, it is an object of the present invention to provide a lateral-flow test device yielding test results with improved validity. In more detail, an object of the present invention is a lateral test device providing improved convenience and reliability with regard to appropriate result reading times.

### Summary of the invention

The object of the present invention is also solved by a lateral flow test device comprising indication means for the detection and quantification of at least Helicobacter pylori antibodies wherein at least one indication means (101) indicates whether a test result will be conclusive, which indication means is not intended to physically contact the sample and at least one indication means (102) indicates that a test result is ready for read.

In one embodiment of the present invention, the test device further comprises at least one indication means (103) (i.e. a control line region) and/or at least one indication means (104) (i.e. a test line region).

In one embodiment of the present invention, the test device is a rapid-flow immunochromatographic test device.

In one embodiment of the present invention, the indication means (101) indicates whether the test result will be inconclusive due to humidity.

In one embodiment of the present invention, the indication means (101)indicated that a result reading time is terminated.

In one embodiment of the present invention, the indication means (101) and (102) are realized by one single indication means.

In one embodiment of the present invention, the indication means (101) and/or (102) comprise at least one humidity indicator material changing color upon contact with water molecules.

In one embodiment of the present invention, the indication means (101) and/or (102) additionally comprise at least one additive intensifying color change as a component of the humidity indication material.

In one embodiment of the present invention, the humidity indicator material changes color when relative humidity of the atmosphere exceeds 30%, 40%, 50%, and /or 60%.

In one preferred embodiment of the present invention, the humidity indicator material changes color when relative humidity of the atmosphere exceeds 40%.

In one embodiment of the present invention, the humidity indicator material changes color within the first hour after being exposed to relative humidity of more than 30%, 40%, 50%, and/or 60%.

In one preferred embodiment of the present invention, the humidity indicator material changes color within the first hour after being exposed to relative humidity of more than 40%.

In one embodiment of the present invention, the humidity indicator material is selected from the group comprising cobalt chloride, copper chloride, lead iodide and/or other indicating compounds.

In one embodiment of the present invention, the test device comprises indication means for detection and quantification of at least one further analyte selected from the group comprising human chorionic gonadotropin (hCG) antigen, *Helcicobacter pylori* antibody, HIV antibody, human lutropin (hLH), human follitropin (hFSH), C-reactive protein, thyroid stimulating hormone (TSH), influenza A virus, influenza B virus, adenovirus, rotavirus, creatine kinase MB isoenzyme (CK-MB), myoglobin, *Chlamydia,* malaria pathogen, prostate-specific antigen (PSA), alpha-fetoprotein (AFP), carcinoembryonic antigen (CEA), tuberculosis (TB) pathogen, *Brucella,* troponin C, troponin I, troponin T, *Toxoplasma,* rubella virus, *Salmonella,* Pan *Salmonella, Listeria,* marihuana, methamphetamine, morphine, amphetamine, cocaine, phencyclidine, barbital, methadone, oxycodone, ethadone, etc.

In one embodiment of the present invention, the analyte is present in bodily fluid selected from the group comprising urine, whole blood, serum, plasma, sperm, menstrual fluid, amniotic fluid, spinal fluid, and synovial fluid.

In one embodiment of the present invention, the analyte is present in solutions, suspensions or dispersions selected from the group comprising cell culture supernatant, cellular extract and tissue extract.

In one embodiment of the present invention, the analyte is present in synthetic solutions and extracts selected from the group comprising environmentral samples, natural and indusrial products.

In one embodiment of the present invention, the test device further comprises a second indication means (102) indicating that a test result is ready for read and/or indicating that a result reading time is terminated.

In one embodiment of the present invention, the indicator material comprised by the second indication means (102) is selected from a solvatochromic compound, a pH indicator, a chromoreactant and mixtures thereof.

In one embodiment of the present invention, the test device is in the form of a test strip.

The application furthermore describes a procedure of manufacture of a lateral-flow test device comprising the steps of:
(1) preparing at least one humidity indication material;
(2) providing indication means (101) and/or (102) with said humidity indication material.

The humidity indication material used in the procedure of manufacture may be selected from the group comprising cobalt chloride, copper chloride and lead iodide compounds.

The procedure of manufacture may additionally comprises the steps of:
(1) Preparing at least one gold releasing pad, comprising the steps of:
   (a) preparing a 1% gold chloride solution;
   (b) preparing a 4% trisodium citrate solution and/or another reducing solution according to the colloidal gold solution particle size;
   (c) preparing a colloidal gold solution;
   (d) preparing a 1% potassium carbonate solution;
   (e) preparing a 1% polyethylene glycol solution;
   (f) preparing a stabilizing buffer;
   (g) preparing a borate buffer;
   (h) preparing a sucrose solution.;
   (i) conjugating a specific antibody or antigen with the colloidal gold in solution of step (c);
   (j) soaking a fiber releasing pad into the conjugate solution of step (i) and drying;
   (k) cutting sheets into 8 mm width strips.
(2) Preparing at least one antibody and/or antigen printed nitrocellulose membrane, comprising the steps of.
   (a) preparing a suitable antibody and/or antigen diluting buffer;
   (b) spray printing antibodies and/or antigens onto a nitrocellulose membrane and drying;
   (c) preparing a nitrocellulose membrane blocking solution;
   (d) blocking of the nitrocellulose membrane and drying.
(3) Preparing at least one sample pad, comprising the steps of:
   (a) preparing a suitable sample pad buffer;
   (b) soaking a sample pad into the buffer of step (a) and drying.
(4) Preparing at least one absorbent indication pad, comprising the step of laminating strips of an indication card material onto a pad.
(5) Laminating and cutting, comprising the steps of:
   (a) laminating the antibody and/or antigen printed nitrocellulose membrane onto a vinyl backing;
   (b) laminating a gold conjugate strip;
   (c) laminating an absorbent pad;
   (d) laminating a sample pad;
   (e) cutting a laminated card into strips of suitable width depending on a plastic housing and test format.
(6) Assembling, comprising the steps of:
   (a) assembling the strips of step (5e) and a sample filter into the bottom part of a plastic housing;
   (b) laminating of an atmospheric humidity indication piece onto the inside of the top part of the plastic housing;
   (c) joining the top and bottom parts of the plastic housing;
   (d) joining the cap;
   (e) inserting the test into the pouch with a desiccant and sealing the pouch.

The preparation of an absorbent indication pad of step (4) above may alternatively comprise an application a humidity indicator material solution by impregnating the absorbent pad with the appropriate chemical solution or by spraying this solution onto the pad.

The application also describes a method of varying the time after which an indication means (102) comprised by the lateral-flow test device according the present invention indicates that a test result is ready for read and/or indicates that a result reading time is terminated.

In one embodiment, the variation in time is in the range of about 1 to about 60 minutes.

The method of varying the result reading time may comprises the use of different concentrations of humidity indicator material.

Alternatively, the method of varying the result reading time comprises the use of different concentrations of additive intensifying colour change.

The term "indication means" as used herein may refer to means suitable for indicating the detection of an analyte, e.g. by indicating an analysis reaction product. Such indication may be mediated optically or acoustically, via radioactivity or fluorescence.

The term "humidity indicator material" changing color upon contact with water molecules comprises inorganic compounds which have long been known for quantitatively detecting humidity. Such indicator material has found application also in the medical field where changes in relative humidity are of critical importance. It allows visual inspection of the condition of a sealed package by readily detecting any change in the relative humidity. However, further physical or chemical methods for detecting and indicating humidity are considered.

Examples of inorganic compounds serving as humidity indicators are cobalt chloride, copper chloride or lead iodide compounds. Cobalt(II)chloride forms hydrates of different colors ranging between blue-violet (CoCl₂ · H₂O) and pink (CoCl₂ · 6H₂O). Copper chloride changes color from brown to azure in the presence of water molecules. Solutions of KPbI₃ have been used to impregnate filter paper for detecting traces of water since the complex is split in the presence of water leading to gold-colored PbI₂.

For realizing the present invention, the humidity indicator material can be applied in form of a humidity indication spot on special cards and/or by impregnating of the absorbent pad with an appropriate chemical solution and/or spraying of the chemical solution onto the absorbent pad.

The sensitivity of humidity indication may be varied by applying different amounts of humidity indication material and different concentrations of its solution, respectively. Furthermore, the kind of humidity indication material may be selected according to the preferred sensitivity. Also the thickness and location of the special card may be selected with regard to the preferred sensitivity.

The indication that a test result is ready for read can be managed by variations in volume and properties of the sample to be tested. Furthermore, a gold releasing pad may be used and its material, location and length may be varied. When using nitrocellulose membrane, a blocking procedure may be applied. Also variations in the material, location and length of the sample pad, the absorbent pad or the membrane may be employed.

The term "ready for read" refers to the time following the completion of an analysis reaction but prior to disintegration of the reaction products.

### Brief description of the figures

*Figure 1* shows top and side views of a typical rapid-flow immunochromatographic test device comprising a sample pad (1), a conjugate pad (2), a capture (test) zone (3), e.g. a membrane that incorporates at least one capture reagent, a control (negative) zone (4), an absorbent pad (5), an adhesive (6) and a plastic backing (7).

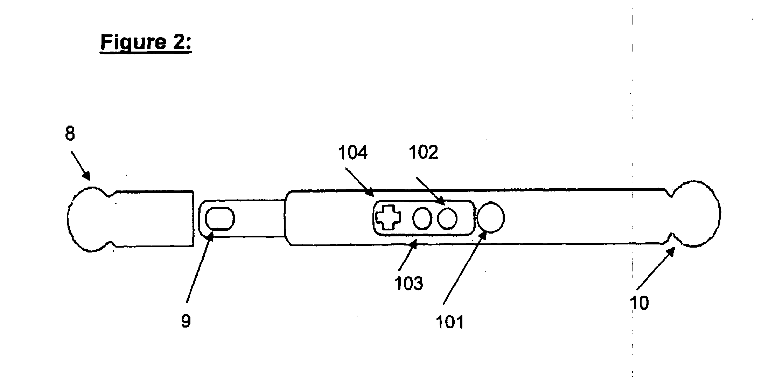

*Figure 2* shows a preferred embodiment of a lateral-flow test device of the present invention comprising a removable cap (8), a sample application window (9), a handle (10), and indication means, namely:
   an indication means (101) realized by a humidity indication hole;
   an indication means (102) realized by a result reading time indication hole;
   an indication means (103) realized by a control line region;
   an indication means (104) realized by a test line region.
*Figure 3* demonstrates the operation of a lateral-flow test device as shown *Figure* 2 with indication means (201) and (202) comprising cobalt chloride as the humidity indicator, and further with indication means (203) and (204).
*Fi-gure 4* demonstrates the operation of a lateral flow test device as shown *Figure* 2 with indication means (301) and (302) comprising copper chloride as the humidity indicator, and further with indication means (303) and (304).

### Detailed description of the invention

### Examples

### Example 1: Operation of a test device of the present invention using cobalt chloride

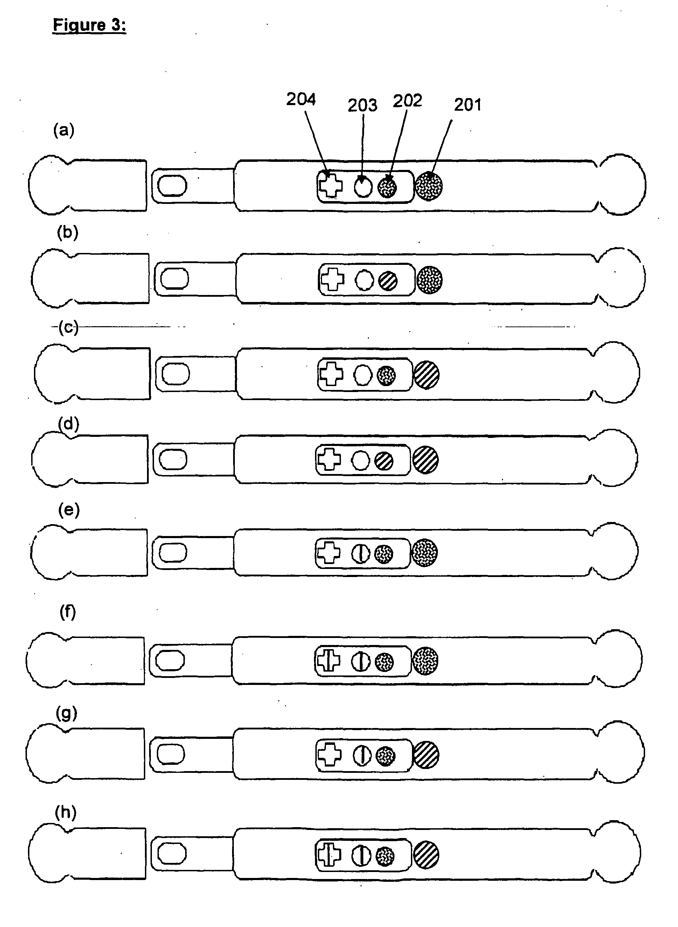

It is referred to *Figure 3*. Both indication means (201) (= humidity indication hole) and indication means (202) (= result reading time indication hole) comprise cobalt chloride as the indicator material, optionally with other additives to meet the intensified color change requirements. Indication means (202) together with indication means (203) (= control line region) and (204) (= test line region) are constructed such that they get in contact with a sample applied to the sample application window. In contrast, means (201) is not intended to physically contact the sample, e.g. by fluid communication. As a consequence, indication means (201) and indication means (202) are capable of indicating high relative atmospheric humidity, e.g. more than 40%, whereas only indication means (202) is capable of indicating that the sample has passed indication means (204) and (203), so that the reaction is completed and-thus the test result is ready for read.

The information provided by a cobalt chloride test device as described is drawn up below (see also *Figure 3*, wherein "pink" is represented by a dotted region, "blue" is represented by a diagonally hatched region, and the "pink/purple band" is represented by a black band).

| | | |
|---|---|---|
| (a) | Indication means (201): | pink |
| | Indication means (202): | pink |
| | Result: | **inconclusive** |
| | Comment: | The test must be repeated with new device. |
| (b) | Indication means (201): | pink |
| | Indication means (202): | blue |
| | Result: | **inconclusive** |
| | Comment: | The test must be repeated with new device. |
| (c) | Indication means (201): | blue |
| | Indication means (202): | pink |
| | Result: | **inconclusive** |
| | Comment: | The test must be repeated with new device. |
| (d) | Indication means (201): | blue |
| | Indication means (202): | blue |
| | Result: | **conclusive** |
| | Comment: | The test device can be safely used. |
| (e) | Indication means (201): | pink |
| | Indication means (202): | pink |
| | Indication means (203): | pink/purple band |
| | Indication means (204): | blank |
| | Result: | **negative** |
| (f) | Indication means (201): | pink |
| | Indication means (202): | pink |
| | Indication means (203): | pink/purple band |
| | Indication means (204): | pink/purple band |
| | Result: | **positive** |
| (g) | Indication means (201): | blue |
| | Indication means (202): | pink |
| | Indication means (203): | pink/purple band |
| | Indication means (204): | blank |
| | Result: | **negative** |
| (h) | Indication means (201): | blue |
| | Indication means (202): | pink |
| | Indication means (203): | pink/purple band |
| | Indication means (204): | pink/purple band |
| | Result: | **positive** |

### Example 2: Operation of a test device of the present invention using copper chloride.

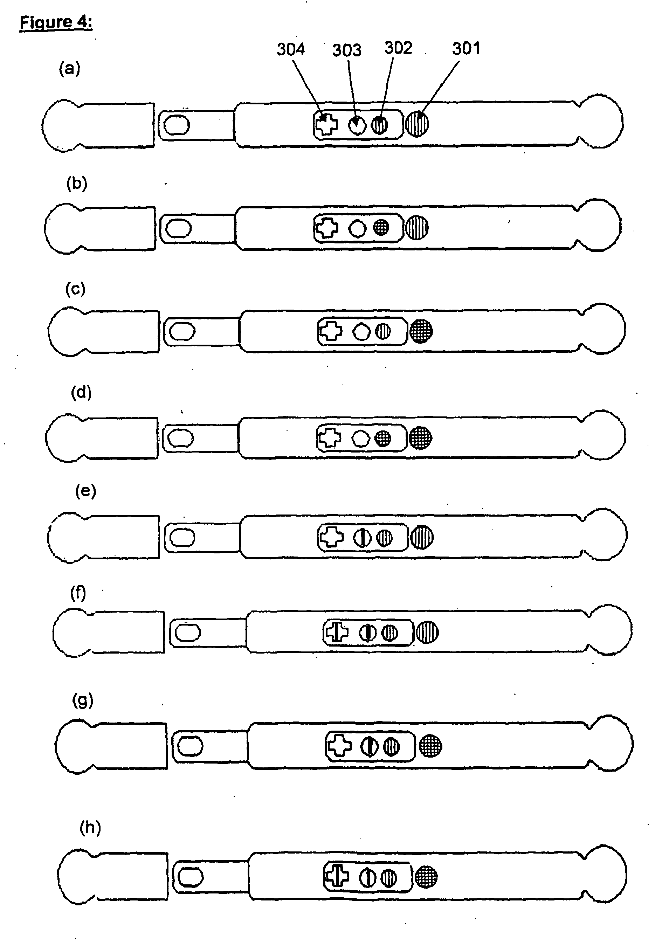

It is referred to *Figure 4**.* The indication means are identified as follows: (301) = humidity indication hole; (302) = result reading time indication hole; (303) = control line region; and (304) = test line region. In contrast to the test device of Example 1, this test device comprises copper chloride instead of cobalt chloride as the humidity indicator material.

The information provided by a copper chloride test device as described is drawn up below (see also *Figure 4*, wherein "azure" is represented a vertically hatched region, "brown" is represented by a squared region, and the "pink/purple band" is represented by a black band).

| | | |
|---|---|---|
| (a) | Indication means (301): | azure |
| | Indication means (302): | azure |
| | Result: | **inconclusive** |
| | Comment: | The test must be repeated with new device. |
| (b) | Indication means (301): | azure |
| | Indication means (302): | brown |
| | Result: | **inconclusive** |
| | Comment: | The test must be repeated with new device. |
| (c) | Indication means (301): | brown |
| | Indication means (302): | azure |
| | Result: | **inconclusive** |
| | Comment: | The test must be repeated with new device. |
| (d) | Indication means (301): | brown |
| | Indication means (302): | brown |
| | Result: | **conclusive** |
| | Comment: | The test device can be safely used. |
| (e) | Indication means (301): | azure |
| | Indication means (302): | azure |
| | Indication means (303): | pink/purple band |
| | Indication means (304): | blank |
| | Result: | **negative** |
| (f) | Indication means (301): | azure |
| | Indication means (302): | azure |
| | Indication means (303): | pink/purple band |
| | Indication means (304): | pink/purple band |
| | Result: | **positive** |
| (g) | Indication means (301): | brown |
| | Indication means (302): | azure |
| | Indication means (303): | pink/purple band |
| | Indication means (304): | blank |
| | Result: | **negative** |
| (h) | Indication means (301): | brown |
| | Indication means (302): | azure |
| | Indication means (303): | pink/purple band |
| | Indication means (304): | pink/purple band |
| | Result: | **positive** |

### Example 3: Use of a test device of the present invention for hCG antigen detection

Colour changes within 1-2 minutes by considering the following parameters:
immerging an absorbent pad with 40% humidity indicator reagent (or a layer of three 40% spots);
high flow rate system components by using low capillary rise of a nitrocellulose membrane, enough sample volume, etc.;
high concentration of printed antibodies.

### Example 4: Use of a test device of the present invention for H. pylori antigen detection

Colour changes within 10 minutes by considering the following parameters:
immerging an absorbent pad with 60% humidity indicator reagent (or 40% spot); low flow rate system components by using a suitable nitrocellulose membrane of high capillary rise, small buffer volume, etc.

### Example 5: Use of a test device of the present invention for HIV antibody detection

Colour changes within 15 minutes by considering the following parameters:
immerging an absorbent pad with 60% humidity indicator reagent (or 50% spot);
very slow flow rate system components by using a suitable nitrocellulose membrane of higher capillary rise, smaller buffer volume, etc.

### Example 6: Test device providing variable result reading times

The indication means (202) (= result reading time indication hole) and the test device can be configured in many different ways allowing maximum variability of result reading times. Different result reading times can be achieved by different concentrations and compositions of the indicator material as well as by different formats of the result reading time indication hole and of the test device itself. The use of different configurations allows the design of highly flexible detection systems that are able to measure variable result reading times, e.g. in the range from 1 minute up to more than 30 minutes, preferably up to more than 60 minutes. For example, the result reading time can be 1, 5 10, 15, 20; 30, 45 or 60 minutes, but any other time between 1 minute and 60 minutes is considered as well. The need of maximum flexibility in result reading times derives from the variety in the nature of assay reactions requiring different reaction times.

Different concentrations of the indicator material will result in different times of response, i.e. low concentrations will respond quickly to humidity while slow responses will be obtained with high concentrations. By varying the concentrations of the indicator material, the desired relative humidity for starting the colour change can be selected.

In addition to the true indicator compound(s), an indicator material can comprise a variety of additives. Hygroscopic additives, for example, can modulate the indicator's sensitivity to humidity. Such a hygroscopic additive can be one that intensifies the colour change. Other additives diminishing the intensity are also considered. By managing the kind and compositions of such additives as well as their concentrations, the time of colour change can be varied.

Different formats of the result reading time means can be realized, for example, by one or more layers, e.g. pad(s), placed onto the test device or by a solution containing cavity. Furthermore, the components of a lateral immunochromatographic test device (e.g. sample pad, gold releasing pad, absorbent pad, nitrocellulose membrane and blood filter or wick) having different dimensions and properties affect the time needed by the sample to reach the indicator material that may be provided within an absorbent pad. By managing these components, the time after which the indicator colour will change can be varied.

The properties and quantities of a sample will also affect its flow rate and thus the time needed for reaching an optional absorbent pad and the indicator material.

### Example 7: Test device providing variability of result reading time termination

Optionally, the indicator substance has to be washed (faded) within a suitable time of colour change in order to indicate that the time for reading results has been terminated ("Do not read results after colour disappearance"). This indication function thus provides another internal timer function which can be used together with the timer functions described in Example 6 or can be used separately.

Similar to the strategies of managing the onset of the result reading time (as outlined in Example 6), different concentrations of the indicator material, different additives and additive concentrations and different formats will be used in order to vary the fading time. Low concentrations of indicator material will be washed quicker than high concentrations. Fixation additives, for example, will affect the time for the indicator washing. By managing the kind, compositions and concentrations of additives, the time of washing (changing) the colour can be varied.

### Example 8: Test device providing more than one result reading time functions

Optionally, a second timer function can be used, wherein the second timer indication material can be a solvatochromic compound, a pH indicator or a chromoreactant or a mixture thereof. The second timer function can be located in the same region as the first timer function or can be located in a separated region. In the case of two timer functions in a single region, a mixture of two or more indicator compounds are used. The second internal timer signal can be realised by appearance of a new colour, by colour change or by colour disappearance

### References

(1) J Chandler, N Robinson, and K Whiting, "Handling False Signals in Gold-Based Rapid Tests" - IVD Technology 7, no. 2 (2001): 34-45; http://www.devicelink.comlivdtlarchive/01/03/002.html
(2) J Chandler, T Gurmin, and N Robinson, "The Place of Gold in Rapid Tests" - IVD Technology 6, no. 2 (2000): 37-49; http://www.devicelink.com/ivdt/archive/00/03/004.html
(3) TC Tisone et al., "Image Analysis for Rapid-Flow Diagnostics" - IVD Technology 5, no. 5 (1999): 52-58; http://www.devicelink.com/ivdt/archive/99/09/010.html

## Claims

1. A lateral-flow test device comprising indication means for detection and quantification of at least *Helicobacter pylori* antibody, wherein at least one indication means (101) indicates whether a test result will be inconclusive due to humidity, which indication means is not intended to physically contact the sample, and at least one further indication means (102) indicates that a test result is ready for read.

2. The test device according to claim 1, further comprising at least one control line region (103) and/or at least one test line region (104).

3. The test device according to claim 1 or 2, wherein the test device is a rapid-flow immunochromatographic test device.

4. The test device according to any of the preceding claims, wherein the indication means (102) indicates that a result reading time is terminated.

5. The test device according to any of the preceding claims, wherein the indication means (101) and/or (102) comprise at least one humidity indicator material changing colour upon contact with water molecules.

6. The test device according to any of the preceding claims, wherein the indication means (101) and/or (102) additionally comprise at least one additive intensifying colour change.

7. The test device according to any of the preceding claims, wherein the humidity indicator material changes colour when relative humidity of the atmosphere exceeds 40%.

8. The test device according to claim 7, wherein the humidity indicator material changes colour within the first hour after being exposed to relative humidity of more than 40%.

9. The test device according to any of the preceding claims, wherein the humidity indicator material is selected from the group comprising cobalt chloride, copper chloride and lead iodide compounds.

10. The test device according to any of the preceding claims, wherein the analyte is present in bodily fluid selected from the group comprising urine, whole blood, serum, plasma, sperm, menstrual fluid, amniotic fluid, spinal fluid and synovial fluid.

11. The test device according to any of the preceding claims, wherein the analyte is present in solutions, suspensions or dispersions selected from the group comprising cell culture supernatant, cellular extract and tissue extract.

12. The test device according to any of the preceding claims, further comprising a second indication means (102) indicating that a test result is ready for read and/or indicating that a result reading time is terminated.

13. The test device according to claim 12, wherein the indicator material is selected from a solvatochromic compound, a pH indicator, a chromoreactant and mixtures thereof.

14. The test device according to any of the preceding claims, wherein the test device is in the form of a test strip.

15. A use of a lateral-flow test device for detection and quantification of at least *Helicobacter pylori* antibody, the test device comprising indication means, wherein at least one indication means (101) indicates whether a test result will be inconclusive due to humidity, which indication means is not intended to physically contact the sample, and at least one indication means (102) indicates that a test result is ready for read.

## Patentansprüche

1. Lateralfluss-Testvorrichtung, die Anzeigemittel zum Nachweis und zur Quantifizierung von mindestens *Helicobacter pylori*-Antikörper umfasst, wobei mindestens ein Anzeigemittel (101) anzeigt, ob ein Testergebnis aufgrund von Feuchtigkeit nicht schlüssig sein wird, wobei das Anzeigemittel nicht dazu vorgesehen ist, mit der Probe physisch in Kontakt zu treten, und mindestens ein weiteres Anzeigemittel (102) anzeigt, dass ein Testergebnis fertig ist, um abgelesen zu werden.

2. Testvorrichtung nach Anspruch 1, die weiterhin mindestens einen Bereich mit einer Kontrolllinie (103) und/oder mindestens einen Bereich mit einer Testlinie (104) umfasst.

3. Testvorrichtung nach Anspruch 1 oder 2, wobei die Testvorrichtung eine immunchromatographische "rapid-flow"-Testvorrichtung ist.

4. Testvorrichtung nach einem der vorangehenden Ansprüche, wobei das Anzeigemittel (102) anzeigt, dass die Zeit zum Ablesen eines Ergebnisses abgelaufen ist.

5. Testvorrichtung nach einem der vorangehenden Ansprüche, wobei das Anzeigemittel (101) und/oder (102) mindestens ein Feuchtigkeitsindikatormaterial umfasst, das die Farbe nach Kontakt mit Wassermolekülen ändert.

6. Testvorrichtung nach einem der vorangehenden Ansprüche, wobei das Anzeigemittel (101) und/oder (102) zusätzlich mindestens ein Additiv umfasst, das die Farbänderung intensiviert.

7. Testvorrichtung nach einem der vorangehenden Ansprüche, wobei das Feuchtigkeitsindikatormaterial die Farbe ändert, wenn die relative Feuchtigkeit der Atmosphäre 40 % übersteigt.

8. Testvorrichtung nach Anspruch 7, wobei das Feuchtigkeitsindikatormaterial die Farbe innerhalb der ersten Stunde ändert, nachdem es einer relativen Feuchtigkeit von mehr als 40 % ausgesetzt worden ist.

9. Testvorrichtung nach einem der vorangehenden Ansprüche, wobei das Feuchtigkeitsindikatormaterial ausgewählt ist aus der Gruppe, umfassend Kobaltchlorid-, Kupferchlorid- und Bleijodid-Verbindungen.

10. Testvorrichtung nach einem der vorangehenden Ansprüche, wobei der Analyt in Körperflüssigkeit vorhanden ist, die ausgewählt ist aus der Gruppe, umfassend Urin, Vollblut, Serum, Plasma, Sperma, Menstruationsflüssigkeit, Amnionflüssigkeit, Spinalflüssigkeit und Synovialflüssigkeit.

11. Testvorrichtung nach einem der vorangehenden Ansprüche, wobei der Analyt in Lösungen, Suspensionen oder Dispersionen vorhanden ist, die ausgewählt sind aus der Gruppe, umfassend Zellkulturüberstand, Zellextrakt und Gewebeextrakt.

12. Testvorrichtung nach einem der vorangehenden Ansprüche, die weiterhin ein zweites Anzeigemittel (102) umfasst, das anzeigt, dass ein Testergebnis fertig ist, um abgelesen zu werden, und/oder anzeigt, dass die Zeit zum Ablesen eines Ergebnisses abgelaufen ist.

13. Testvorrichtung nach Anspruch 12, wobei das Indikatormaterial ausgewählt ist aus einer solvatochromen Verbindung, einem pH-Indikator, einem Chromoreaktionspartner und Mischungen davon.

14. Testvorrichtung nach einem der vorangehenden Ansprüche, wobei die Testvorrichtung in Form eines Teststreifens vorliegt.

15. Verwendung einer Lateralfluss-Testvorrichtung zum Nachweis und zur Quantifizierung von mindestens *Helicobacter pylori*-Antikörper, wobei die Testvorrichtung Anzeigemittel umfasst, wobei mindestens ein Anzeigemittel (101) anzeigt, ob ein Testergebnis aufgrund von Feuchtigkeit nicht schlüssig sein wird, wobei das Anzeigemittel nicht dazu vorgesehen ist, mit der Probe physisch in Kontakt zu treten, und mindestens ein Anzeigemittel (102) anzeigt, dass ein Testergebnis fertig ist, um abgelesen zu werden.

## Revendications

1. Dispositif de test à flux latéral comprenant des moyens d'indication pour la détection et la quantification d'au moins un anticorps de *Helicobacter pylori,* dans lequel au moins un moyen d'indication (101) indique si un résultat de test sera non concluant à cause de l'humidité, lequel moyen d'indication n'est pas prévu entrer en contact physique avec l'échantillon, et au moins un autre moyen d'indication (102) indique qu'un résultat de test est prêt à lire.

2. Dispositif de test selon la revendication 1, comprenant en outre au moins une région de ligne de contrôle (103) et/ou au moins une région de ligne de test (104).

3. Dispositif de test selon la revendication 1 ou 2, où le dispositif de test est un dispositif de test immunochromatographique à flux rapide.

4. Dispositif de test selon l'une quelconque des revendications précédentes, dans lequel le moyen d'indication (102) indique qu'un temps de lecture de résultat est terminé.

5. Dispositif de test selon l'une quelconque des revendications précédentes, dans lequel le moyen d'indication (101) et/ou (102) comprend au moins un matériau indicateur de l'humidité changeant la couleur après contact avec des molécules d'eau.

6. Dispositif de test selon l'une quelconque des revendications précédentes, dans lequel le moyen d'indication (101) et/ou (102) comprend en outre au moins un additif intensifiant le changement de la couleur.

7. Dispositif de test selon l'une quelconque des revendications précédentes, dans lequel le matériau indicateur de l'humidité change la couleur lorsque l'humidité relative de l'atmosphère dépasse 40 %.

8. Dispositif de test selon la revendication 7, dans lequel le matériau indicateur de l'humidité change la couleur au cours de la première heure après l'exposition à une humidité relative supérieure à 40 %.

9. Dispositif de test selon l'une quelconque des revendications précédentes, dans lequel le matériau indicateur de l'humidité est choisi dans le groupe comprenant des composés de chlorure de cobalt, de chlorure de cuivre et d'iodure de plomb.

10. Dispositif de test selon l'une quelconque des revendications précédentes, dans lequel l'analyte est présent dans le liquide corporel choisi dans le groupe comprenant l'urine, le sang total, le sérum, le plasma, le sperme, le liquide menstruel, le liquide amniotique, le liquide céphalorachidien et le liquide synovial.

11. Dispositif de test selon l'une quelconque des revendications précédentes, dans lequel l'analyte est présent dans des solutions, des suspensions ou des dispersions choisies dans le groupe comprenant du surnageant de culture cellulaire, de l'extrait cellulaire et de l'extrait tissulaire.

12. Dispositif de test selon l'une quelconque des revendications précédentes, comprenant en outre un second moyen d'indication (102) indiquant qu'un résultat de test est prêt à lire et/ou indiquant qu'un temps de lecture de résultat est terminé.

13. Dispositif de test selon la revendication 12, dans lequel le matériau indicateur est choisi parmi un composé solvatochromique, un indicateur de pH, un réactif à base de chrome et des mélanges de ceux-ci.

14. Dispositif de test selon l'une quelconque des revendications précédentes, où le dispositif de test est sous la forme d'une bandelette de test.

15. Utilisation d'un dispositif de test à flux latéral pour la détection et la quantification d'au moins un anticorps de *Helicobacter pylori,* le dispositif de test comprenant des moyens d'indication, où au moins un moyen d'indication (101) indique si un résultat de test sera non concluant à cause de l'humidité, lequel moyen d'indication n'est pas prévu entrer en contact physique avec l'échantillon, et au moins un moyen d'indication (102) indique qu'un résultat de test est prêt à lire.
